# EUROPEAN PATENT SPECIFICATION

(11) **EP 4 180 081 B1**
(45) Date of publication and mention of the grant of the patent: **08.04.2026**
(21) Application number: 21837908.9
(22) Date of filing: 07.07.2021
(51) Int. Cl.: A61M 25/02, A61B 5/25, G01N 27/02

(54) **BIOCONTACT DETECTION SENSOR AND BIOCONTACT DETECTION DEVICE USING SAME**
BIOKONTAKTDETEKTIONSSENSOR UND BIOKONTAKTDETEKTIONSVORRICHTUNG DAMIT
CAPTEUR DE DÉTECTION DE BIOCONTACT ET DISPOSITIF DE DÉTECTION DE BIOCONTACT L'UTILISANT

(30) Priority: 08.07.2020 JP 2020118115
(43) Date of publication of application: 17.05.2023
(73) Proprietor: National University Corporation Chiba University, Chiba-shi, Chiba 263-8522 (JP); I Medex Co., Ltd., Chiba-shi, Chiba 262-0003 (JP)
(72) Inventor: YAMASHITA Ayumi, Chiba-shi, Chiba 260-8672 (JP); SUGAWARA Hisayoshi, Chiba-shi, Chiba 260-8672 (JP); ICHIDA Makoto, Chiba-shi, Chiba 262-0003 (JP); MINOWA Takashiro, Chiba-shi, Chiba 262-0003 (JP); SUGASAWA Yasuhisa, Chiba-shi, Chiba 262-0003 (JP)
(74) Representative: Lavoix
(86) International application number: PCT/JP2021/025699
(87) International publication number: WO 2022/009941

(56) References cited:
- WO-A1-2019/020666
- JP-A- 2007 020 801
- JP-A- 2007 020 801
- US-A1- 2020 008 299

## Description

### Technical Field

The present disclosure relates to a sensor for detecting contact of a living body, more particularly to a living-body contact detection sensor for detecting contact for removing or peeling by a wearer, and to a living-body contact detection device using the living-body contact detection sensor.

### Background Art

In a medical facility such as a hospital, a medical catheter is indwelled in a patient for treatment at the time of extracorporeal circulation in artificial dialysis, supply of a medicinal solution by drip infusion, and the like. In addition, bandages, covering materials (including "skin bonding tape", the same applying hereinafter), and the like are applied to patients and the like after surgical operations in order to protect wounds. However, there are various patients who receive such treatment, and in a patient with severe and mild consciousness disorder, a patient with dementia, or a patient with insufficient understanding of a therapeutic instrument and a use method, the patient may remove the medical catheter or remove a bandage, a covering material, and the like.

Therefore, conventionally, some techniques for detecting a removal of the medical catheter have been proposed. For example, Patent Literature 1 (JP H05-79468 U) proposes a flexible liquid leakage detection device in which two or more foil-shaped electrodes are sandwiched between a synthetic resin tape and a synthetic resin nonwoven fabric tape, a sensor provided with an adhesive layer on a surface of the nonwoven fabric tape is attached in the vicinity of an indwelling needle, and a drip liquid or blood is detected by a short circuit between the electrodes when the leaked drip liquid or blood comes into contact with the electrode through the nonwoven fabric tape to indirectly detect the dropping of the indwelling needle.

In addition, as a technique for detecting a sign leading to removal of a drip needle before the drip needle is removed, Patent Literature 2 (JP 2015-66071 A) proposes a monitoring device including: a first detection unit configured to detect a state of a place where a drip needle has been inserted into a monitoring target person based on an image obtained by photographing the monitoring target person; a second detection unit configured to detect a state of a tube connected to the drip needle based on the image; a determination unit that determines whether there is a possibility of removal of the drip needle based on a change in at least one of the detected state of the place where the drip needle has been inserted and the detected state of the tube; and an output unit that outputs an alarm indicating the possibility of removal of the drip needle when it is determined that there is a possibility of removal of the drip needle. JP 2007 020801 A discloses a monitoring device which comprises a puncture needle capable of puncturing a patient, and a flexible tube extending from the puncture needle capable of introducing a liquid into the patient's body and leading out blood from inside of the patient's body via the puncture needle . The monitoring device is equipped also with an electrode part which is formed on a surface different from a contact face of a wing for attaching and fixing the flexible tube to the patient during treatment and senses that the patient is brought into contact with the wing, and a signal detection means 7 for generating a signal when the contact to the wing is detected by the electrode part.

### Citation List

### Patent Literature

Patent Literature 1: JP H05-79468 U
Patent Literature 2: JP 2015-66071 A
Patent Literature 3: JP 2007 020801 A

### Summary of Invention

### Technical Problem

With the flexible liquid leakage detection device according to Patent Literature 1, it is possible to detect a removal of an indwelling needle by the leaked drip liquid or blood, but it is difficult to prevent the needle removal situation. In addition, in Patent Literature 2, since a possibility of removal is predicted from the state of the place where the drip needle has been inserted and the state of the tube, the needle removal itself can be prevented, but it is a prediction after all and there is still room for improvement in accuracy.

Therefore, a first object of the present disclosure is to provide a living-body contact detection sensor capable of detecting in advance that a patient removes a medical catheter or removes a bandage, a covering material, a skin bonding tape, and the like, and a living-body contact detection device using the living-body contact detection sensor.

Furthermore, a second object of the present disclosure different from the first object is to provide a living-body contact detection sensor that not only monitors living-body contact with an operation site in a patient but also monitors whether or not a living body is in contact in various applications, and a living-body contact detection device using the living-body contact detection sensor.

### Solution to Problem

The invention is as defined in the appended claims.

The present disclosure provides a living-body contact detection sensor for detecting contact of a living body that includes a base material having a sheet-like shape and a detection unit made of a conductive material provided on one surface or both surfaces of the base material, in which the detection unit includes one or more pairs of wirings or electrodes having different polarities, and the wirings or electrodes are provided such that at least one of a current, a voltage, and an electric resistance is changed by the contact of the living body.

Furthermore, in the living-body contact detection sensor of the present invention, the detection unit includes a pair or more of wirings or electrodes that are not electrically connected to each other, and the wirings or electrodes expose the conductive material and can be formed at intervals at which the wirings or electrodes are electrically short-circuited by the contact of the living body.

Furthermore, in the living-body contact detection sensor of the present invention, the detection unit includes a pair or more of wirings or electrodes that are electrically connected to each other, and the wirings or electrodes expose the conductive material and can be formed at intervals at which at least one of a current, a voltage, and an electric resistance changes by the contact of the living body.

In the living-body contact detection sensor according to the present invention, the detection unit is formed by adjacently arranging or oppositely arranging the one or more pairs of wirings or electrodes having different polarities, and the wirings or electrodes can be formed apart from each other at an interval at which at least one of a current, a voltage, and an electric resistance changes by the contact of the living body. That is, in a case where the one or more pairs of wirings or electrodes are arranged adjacent to each other, at least one of the current, the voltage, and the electric resistance can be changed by energizing via the living body by the contact of the living body, and in a case where the one or more pairs of wirings or electrodes are arranged facing each other, the wirings or electrodes are in contact with each other and energized by the contact of the living body, and at least one of the current, the voltage, and the electric resistance can be changed. In particular, when the wirings or electrodes are arranged adjacent to each other, it is desirable that the wirings or electrodes be configured such that the conductive material can be exposed so as to be energized by the contact of the living body.

The detection unit can adjust the detection sensitivity according to the necessity or urgency of detecting the contact of the living body. The detection sensitivity of the detection unit can be adjusted by the material and thickness of the wirings or electrodes constituting the detection unit, the arrangement interval (distance), or the like. For example, in a case where the contact of the living body with a medical device in which artificial respiration, a nutrition catheter, an arterial line, or the like is indwelled is detected, since urgency of the contact detection is high, the interval between the wirings or electrodes can be set to 1 to 5 mm to increase the sensitivity of the detection unit. In addition, in a case where the contact of the living body with a peripheral indwelling needle, a wound, a urinary catheter, a diaper, or the like is detected, there may be a situation in which the urgency of the contact detection is not high, and therefore, the interval between the wirings or electrodes can be set to 1 to 10 mm, which allows the sensitivity of the detection unit to be lowered.

Furthermore, the detection unit can adjust sensitivity or the like according to a portion to be in contact with the detection unit. For example, in the case of detecting the contact of an entire hand, the detection sensitivity can be lowered by setting the interval between the wirings or electrodes to 5 to 30 mm, and in the case of detecting the contact of a finger or the like, the detection sensitivity can be increased by setting the interval between the wirings or electrodes to 1 to 7 mm. That is, in the living-body contact detection sensor according to the present invention, the sensitivity at the time of the contact of the living body can be adjusted by adjusting the width or arrangement interval of the wirings or electrodes. Then, by setting or adjusting a threshold in a determination module, which will be described later, depending on the detection sensitivity in the detection unit, it is possible to realize a living-body contact detection sensor with less false reporting and less non-reporting.

The base material having a sheet-like shape on which the detection unit is provided can be formed of various materials such as a resin sheet and a fabric, and a material having an electrical insulation property can be used for at least a surface on which the detection unit is provided, in order to prevent a short circuit between the wirings or electrodes constituting the detection unit. In addition, when the living-body contact detection sensor is used by being attached to a patient, the base material may be formed of a sheet material having flexibility. Furthermore, when the wirings or electrodes do not have elasticity, the base material can be formed of a sheet having no elasticity or low elasticity in order to prevent the wirings or electrodes from being cut. In a case where the base material is made of a sheet having stretchability and flexibility, it is possible to follow the bending and stretching operation by a patient that it is attached to, and can reduce discomfort at the time of attachment. On the other hand, as the base material, a sheet that does not expand and contract can be used in consideration of disconnection, and a sheet having flexibility to the extent that the wirings or electrodes are not disconnected can also be used. In particular, when the wirings or electrodes are formed of carbon, it is desirable that the base material is a sheet having strength to such an extent that carbon is not broken. In addition, when the base material is used by being directly attached to the epidermis of a patient, the base material is desirably formed using a biocompatible material, for example, a material that has passed a biocompatibility test based on the ISO standard.

It is desirable to provide an attachment portion on the base material in consideration of ease of attachment to a patient. This is for enhancing versatility by enabling the base material to be attached to various parts. The attachment portion may have a structure in which an adhesive or a hook-and-loop fastener is provided on any surface of the base material, and the base material is wound around and fixed to an affected part. For example, in a case where the living-body contact detection sensor is attached onto a bandage, it is desirable to employ a glue or an adhesive depending on a site to be used, by selecting a material that can be attached onto the bandage, or the like. In addition, it is desirable that the attachment portion is easy to attach and is not able to be easily detached by a patient that it is attached to. In addition, in a case where the attachment portion is not provided, the living-body contact detection sensor can be fixed with a tape, a magnet, or the like.

The living-body contact detection sensor of the present invention can be directly attached to the skin, attached to a medical instrument such as a cannula, or attached to a treatment part in which a medical instrument such as a drip needle is indwelled and is protected with a bandage or the like. In particular, in a case of attaching onto the treatment part protected with a bandage or the like, when a patient touches the living-body contact detection sensor of the present invention, the contact is detected and notified, and then the patient have to remove the bandage or the like, so that it takes time for reaching the treatment part which is not desired to be contacted and removing (or starting the pull out of) a medical instrument such as a drip needle. That is, since it takes time to remove the medical instrument after detecting the contact in the patient, it is possible for a viewer such as a nurse to take a measure, which can prevent the removal operation in advance.

In addition, a detection unit formed of a conductive material is provided on one surface or both surfaces of the base material. The detection unit can include one or more pairs of wirings or electrodes having different polarities, and the wirings or electrodes can be formed by applying or printing a conductive material, such as silver, copper, carbon, or tin oxide, on the base material. As the conductive material, a conductive paste, a conductive paint, and the like can be used. However, it is natural that the wirings or electrodes can be formed by embedding metal conductive wires in the base material. Such a detection unit can be used without particular limitation as long as at least one of a current, a voltage, and an electric resistance due to the contact of the living body changes between one or more pairs of wirings or electrodes having different polarities. In addition, the wirings or electrodes can be formed in a band shape such that wirings or electrodes having different polarities are alternately adjacent to each other.

Even when the wirings or electrodes provided on the base material come into contact with a conductive member (such as a metal bed frame) other than a living body, at least one of the current, the voltage, and the electric resistance of the wirings or electrodes may change. Therefore, in a case where the wirings or electrodes are energized via the living body, it is desirable to provide a shielding member having an insulating property that covers the wirings or electrodes on the wirings or electrodes so that at least one of the current, the voltage, and the electric resistance does not change by the contact of the conductive member. That is, it is possible to have a structure in which it is necessary to come into contact with the living-body contact detection sensor in order for the patient himself/herself to remove the medical catheter or to remove the bandage, the covering material, the skin bonding tape, or the like, and it is necessary to remove the shielding member in order to come into contact with the living-body contact detection sensor.

The shielding member is made of an insulating material covering the detection unit, and can mainly function to prevent a false report when the detection unit comes into contact with a metal portion, and therefore, at least a surface in contact with the detection unit is desirably made of an insulating material. In addition, the shielding member may be rigid or flexible, and is desirably formed in a sheet-like shape, a film-like shape, or a cylindrical shape. Furthermore, the shielding member may be a stocking net (tube bandage), a supporter, a cuff, and the like.

In addition, in order to solve at least one of the above problems, the present invention provides a living-body contact detection device according to claim 6.

The determination module in the living-body contact detection device can be configured to output a living-body contact signal indicating that the contact of the living body has occurred in a case where a change amount and/or a change time of at least one of the current, the voltage, and the electric resistance acquired by the detection unit exceeds a preset threshold value. Therefore, the determination module can hold a threshold value depending on the purpose of use or the structure and characteristics of the living-body contact detection sensor to be used, and can reduce false reporting and non-reporting by setting the threshold value. Such a determination module can be configured using a numerical value operation device.

Furthermore, in order to solve at least one of the above problems, the present invention provides a living-body contact detection method for detecting contact of a living body according to claim 8.

### Advantageous Effects of Invention

A living-body contact detection sensor for detecting contact of a living body according to the present invention includes a base material having a sheet-like shape and a detection unit made of a conductive material provided on one surface or both surfaces of the base material, and the detection unit includes one or more pairs of wirings or electrodes having different polarities, and the wirings or electrodes expose the conductive material and are formed at intervals at which at least one of a current, a voltage, and an electric resistance changes by the contact of the living body, and a living-body contact detection device using the living-body contact detection sensor includes the living-body contact detection sensor and a determination module that determines presence or absence of the contact of the living body based on a change in at least one of the current, the voltage, and the electric resistance acquired by the detection unit.

In the living-body contact detection sensor according to the present invention, in a case where a patient who is a wearer is in contact with the one or more pairs of wirings or electrodes having different polarities at a part of a living body (a hand, an arm, or the like), the presence or absence of the contact of the living body can be determined by a change in at least one of a current passing through the wirings or electrodes, a voltage, and an electric resistance.

Therefore, it is possible to provide a living-body contact detection sensor and a living-body contact detection device using the living-body contact detection sensor that can detect the contact with the detection unit before the patient removes the medical catheter or removes the bandage, the covering material, the skin bonding tape, or the like, and as a result, can detect these actions in advance.

Furthermore, since the living-body contact detection sensor and the living-body contact detection device using the living-body contact detection sensor can detect contact of a living body by a change in at least one of a current, a voltage, and an electric resistance, it is possible to provide a living-body contact detection sensor that can be used in various applications for not only monitoring contact of a living body with an operation site in a patient but also detecting or monitoring whether or not a living body is in contact, and a living-body contact detection device using the living-body contact detection sensor.

### Brief Description of Drawings

Fig. 1 is a perspective view illustrating a living-body contact detection sensor according to a first embodiment.
Fig. 2 is an exploded perspective view illustrating a state in which a shielding member is provided in the living-body contact detection sensor illustrated in Fig. 1.
Fig. 3 is an exploded perspective view illustrating a living-body contact detection sensor according to a second embodiment.
Fig. 4 is an exploded perspective view illustrating a living-body contact detection sensor according to a third embodiment.
Fig. 5 is an exploded perspective view illustrating a living-body contact detection sensor according to a fourth embodiment.
Fig. 6 is a usage state diagram of a body contact detection device configured using a living-body contact detection sensor.
Fig. 7 is a table showing experimental results of Examples.

### Description of Embodiments

Hereinafter, a living-body contact detection sensor 10 according to the present embodiment and a living-body contact detection device 60 using the same will be specifically described with reference to the drawings. In particular, the present embodiment is specifically illustrated as a living-body contact detection sensor 10 and a living-body contact detection device 60 using the same, which can be attached to a patient in a medical facility or the like and can detect in advance when the patient intentionally removes a medical catheter or detaches a bandage, a covering material, a skin bonding tape, or the like.

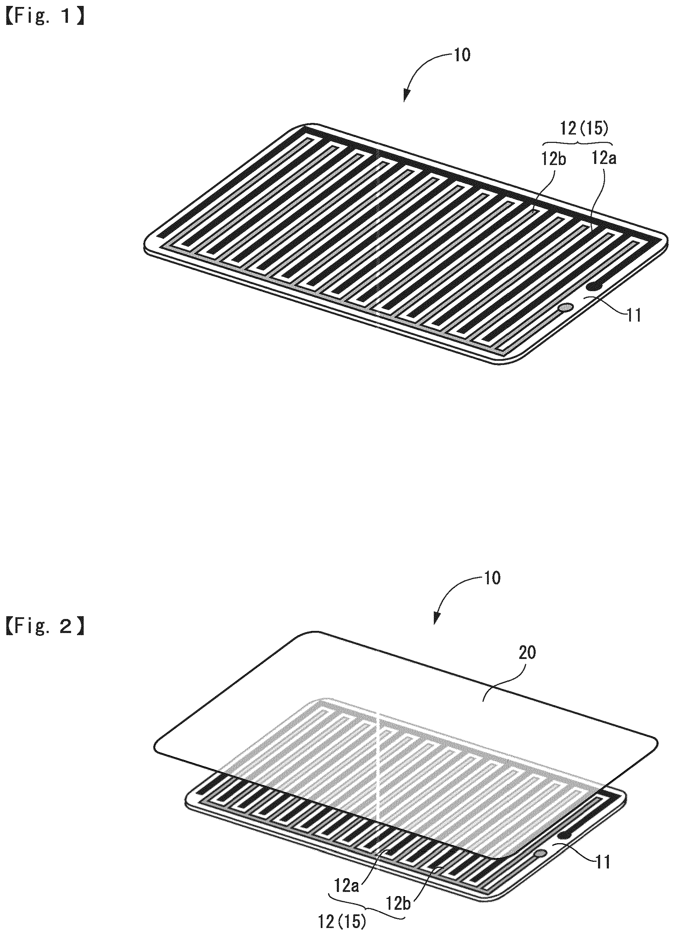

The living-body contact detection sensor 10 according to the first embodiment illustrated in Fig. 1 includes a base material 11 having a sheet-like shape and a detection unit 15 made of a conductive material provided on one side or both sides of the base material 11. In the present embodiment, the base material 11 is formed using a PET sheet having a thickness of 0.075 mm, and is particularly formed in a quadrangular shape. However, the base material 11 can also be formed using another resin material, and can also be formed using fabric. In addition, the shape of the base material 11 can be appropriately changed depending on a portion where the base material 11 is installed, and for example, the base material 11 can be formed in various shapes such as a circular shape and an elliptical shape in addition to being formed in a band shape to be installed so as to be wound around the target portion. Furthermore, the base material 11 has a planar shape, and can also be formed in a three-dimensional shape such as a truncated cone shape.

The base material 11 may be formed using a resin sheet having stretchability and flexibility as long as the wirings or electrodes 12 constituting the detection unit 15 are not disconnected. This is to follow the movement of a user such as a patient wearing this, and to alleviate discomfort at the time of wearing.

The detection unit 15 provided on the base material 11 having a sheet-like shape can be formed of various conductive materials, and carbon is used in the present embodiment. The detection unit 15 can include one or more pairs of wirings or electrodes 12 having different polarities. In the present embodiment, the detection unit 15 is formed of the pair of electrodes 12 including an anode 12a and a cathode 12b. However, a plurality of electrodes 12 including the anode 12a and the cathode 12b may be provided, and the number of electrodes 12 of one polarity may be larger than the number of electrodes 12 of the other polarity. In particular, in the present embodiment, the electrode 12 is formed such that the anode 12a and the cathode 12b formed in a belt shape are alternately adjacent to each other, and each of the anode 12a and the cathode 12b is formed in a comb tooth shape. Providing the electrodes 12 in such a comb shape can uniformly detect the sensitivity at the time of the contact of the living body in any region.

In addition, the wirings or electrodes 12 constituting the detection unit 15 are provided to expose the conductive material. By exposing the conductive material, at least one of the current, the voltage, and the electric resistance is changed by the contact of the living body, and the contact of the living body can be detected by the change amount and the change time. Therefore, it is desirable that the wirings or electrodes 12 having different polarities are formed with a thickness and an interval at which at least one of a current, a voltage, and an electric resistance can be changed by the contact of the living body. In the present embodiment, the detection unit 15 is formed to detect the self-removal operation of the medical catheter, and the detection unit 15 is formed such that the anode 12a and the cathode 12b formed in a band shape are alternately adjacent to each other, and each of the anode 12a and the cathode 12b has a width of 2 to 12 mm and is formed to be separated from each other at an interval of 2 to 12 mm. Since the intentional self-removal operation of the medical catheter is performed by a finger, it is desirable to form the wirings or electrodes 12 having different polarities with a width and an interval with which contact of the finger can be detected.

In particular, in the living-body contact detection sensor 10 according to the present embodiment, the electrodes 12 (the anode 12a and the cathode 12b) can be formed using a conductive material (for example, carbon or the like) in addition to a metal material such as gold, silver, and copper. In particular, in the case of using a material, such as carbon, that can be printed, the electrodes 12 can be formed by printing on the base material 11, and as a result, a manufacturing process and equipment can be simplified, and manufacturing can be performed at low cost. In addition, in a case where the electrodes 12 are formed of metal, the electrode can be stretched by the ductility of the metal itself, and thus, in a case where the base material 11 having flexibility is used, the electrode 12 can be made difficult to be cut.

In the present embodiment, in the detection unit 15, a pair of electrodes 12 having different polarities are arranged without being electrically connected. As a result, when power is supplied to each of the electrodes 12, the electrodes 12 are short-circuited by contact with the living body, which allows electricity to flow. Therefore, by measuring the current value, the presence or absence of the contact of the living body can be monitored.

Since the living-body contact detection sensor 10 formed as described above is provided with the detection unit 15 made of a conductive material on the base material 11 having a sheet-like shape, the living-body contact detection sensor 10 can be directly attached on a living body or provided on a medical holding device such as a bandage or an adhesive tape provided on a living body. That is, since no applicable place is selected, the living-body contact detection sensor 10 has high versatility. In particular, the conductive material is formed such that the wirings or electrodes 12 are exposed, and the contact of the living body is detected by the change in the current, the voltage, and/or the electric resistance due to the contact, so that the operation (contact of the living body) at the time of self-removal can be more accurately detected and prevented as compared with the case of detecting the pressure at the time of contact.

Fig. 2 is an exploded perspective view when a shielding member 20 having a sheet-like shape is laminated on the living-body contact detection sensor 10 illustrated in Fig. 1. If the wirings or electrodes 12 constituting the detection unit 15 are exposed as in the living-body contact detection sensor 10 illustrated in Fig. 1, even in a case where the living-body contact detection sensor comes into contact with a member having conductivity such as a metal bed frame or in a case where fluid such as water or body fluid adheres, the living-body contact detection sensor is energized, and it is therefore concerned that such energization is erroneously detected as contact of a living body. Therefore, in the living-body contact detection sensor 10 according to this embodiment, the shielding member 20 is provided so as to cover at least a portion where the wirings or electrodes 12 are exposed in the detection unit 15.

The shielding member 20 can be formed of a sheet or the like, and can be provided such that the living-body contact detection sensor 10 is not able to be peeled off unless the shielding member 20 is removed. Specifically, a sheet, a film, a bandage, or a fabric can be put on the living-body contact detection sensor 10 attached to a user to cover at least the detection unit 15, or a stockinet, a supporter, a cuff, or the like can be attached on the installed living-body contact detection sensor 10, or the installed living-body contact detection sensor 10 can be covered with clothes (such as sleeves) of an installer.

In a case where the shielding member 20 is provided as described above, in order to remove the medical catheter or to remove a bandage, a covering material, a skin bonding tape, or the like, contact with the detection unit 15 is indispensable in the living-body contact detection sensor 10, and contact with the shielding member 20 is indispensable in order to come into contact with the detection unit 15. Since the wirings or electrodes 12 in the detection unit 15 are covered with the shielding member 20, at least one of the current, the voltage, and the electric resistance in the detection unit 15 does not change unless the patient intentionally peels off the shielding member 20. Therefore, the living-body contact detection sensor 10 that can avoid false reporting and the like is realized.

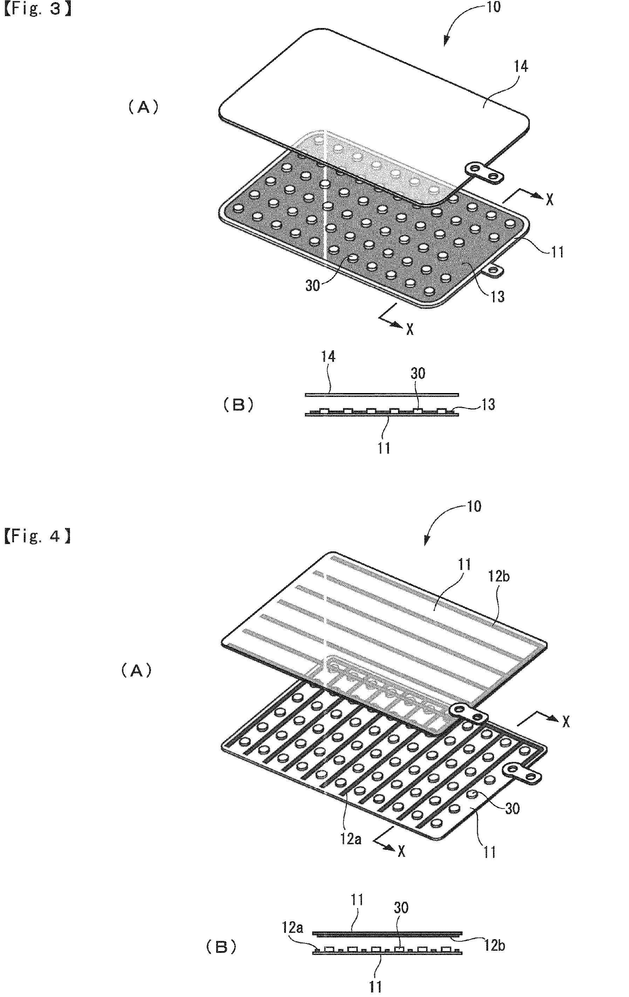

Fig. 3 is an exploded perspective view (A) and a view from the arrow direction of the X-X line (B) of the living-body contact detection sensor 10 according to the second embodiment. In the living-body contact detection sensor 10 according to the second embodiment, a detection unit 15 is configured by arranging one or more pairs of wirings or electrodes 12 having different polarities so as to face each other, and at least one of a current, a voltage, and an electric resistance of the wirings or electrodes 12 is changed by the contact of the living body with the detection unit 15. That is, a pair or more of wirings or electrodes 12 having different polarities are provided on the opposing surfaces of the base materials 11 arranged to face each other, and a spacer is provided which constantly separates the pair or more of wirings or electrodes 12 having different polarities from each other. Then, when the living body comes into contact with the spacer, the spacer is elastically deformed and the wiring or electrode 12 facing the spacer comes into contact with the spacer, so that at least one of the current, the voltage, and the electric resistance can be changed.

As the base material 11, an indium tin oxide (ITO) film 14 can be used, or one in which a conductive material layer 13 such as carbon is provided on the base material 11 can be used. In the indium tin oxide (ITO) film 14, a surface having conductivity can serve as the detection unit 15, and in the base material 11 provided with a conductive material, the conductive material 13 can serve as the detection unit 15. Dot spacers 30 that are elastically deformed by contact with a person and that bring the wirings or electrodes 12 into contact with each other are provided between the detection units (that is, the electrodes 12) arranged to face each other.

In the living-body contact detection sensor 10 according to the present embodiment, carbon is applied to any one of the base materials 11 to form the conductive material layer 13, a plurality of dot spacers 30 made of an elastic resin is provided on the conductive material layer 13 at predetermined intervals, and the ITO film 14 is arranged facing the conductive material layer 13 in a state of being always separated from the conductive material layer 13 by the dot spacers 30.

In the living-body contact detection sensor 10 formed in this manner, at least one of the current, the voltage, and the electric resistance of the detection unit 15 (that is, the wirings or electrodes 12) can be changed by application of certain pressure or more in the thickness direction. In the living-body contact detection sensor 10 according to the second embodiment, the sensitivity at the time of contact can be adjusted by the elasticity, the installation interval, and the installation area of the dot spacers 30.

Fig. 4 is an exploded perspective view (A) and a view from the arrow direction of the X-X line (B) of the living-body contact detection sensor 10 according to the third embodiment. As described in the second embodiment, the living-body contact detection sensor 10 according to the present embodiment is configured such that a pair or more of wirings or electrodes 12 having different polarities are provided on the opposing surface of the base material 11 disposed to face each other, and the dot spacers 30 are provided between the opposing surfaces of both base materials 11. In particular, in the living-body contact detection sensor 10 according to the third embodiment, wirings or electrodes 12 made of a conductive material are formed as the detection unit 15 by printing or the like on each of the base materials 11 arranged facing each other, and the wirings or electrodes 12 provided on any one of the base materials 11 and the wirings or electrodes 12 provided in the other description are formed so as to cross each other. In the present embodiment, in the rectangular base material 11, the wiring or electrode 12a is provided on the lower base material 11 in Fig. 4 so as to extend in the lateral direction, and the wiring or electrode 12b is provided on the upper base material 11 so as to extend in the longitudinal direction. On the opposing surface of the base material 11, the dot spacers 30 using an elastic resin as spacers are provided, and the wirings or electrodes 12 arranged to face each other are always separated by the dot spacers 30. Then, in a case where a predetermined pressure acts in the thickness direction of the living-body contact detection sensor 10, the wirings or electrodes 12 that are always separated come into contact by elastic deformation of the dot spacers 30, and at least one of the current, the voltage, and the electric resistance changes. As a result, the living-body contact detection sensor 10 can detect the presence or absence of contact with the living body.

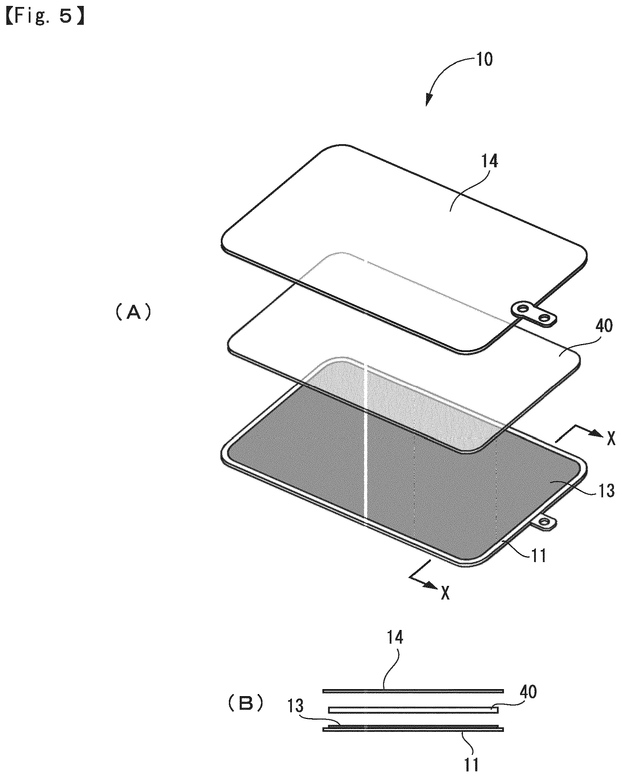

Fig. 5 is an exploded perspective view (A) and a view from the arrow direction of the X-X line (B) of the living-body contact detection sensor 10 according to the fourth embodiment. As described in the third embodiment, the living-body contact detection sensor 10 according to this embodiment is a living-body contact detection sensor 10 in which a conductive gel 40 is provided between the wirings or electrodes 12 arranged facing each other, and one or more pairs of wirings or electrodes 12 having different polarities are separated from each other. As described in the third embodiment, the wirings or electrodes 12 disposed opposite to each other can be realized by using an indium tin oxide (ITO) film 14 as the base material 11, or by using the base material 11 provided with a conductive material layer 13 made of a conductive material such as carbon, or with a wiring or electrode 12. As a conductive gel, an existing conductive gel such as an acrylic gel or a urethane gel can be used.

According to the living-body contact detection sensor 10 configured as described above, in a case where pressure is applied in the thickness direction by contact, at least one of the current, the voltage, and the electric resistance changes by the contact between the wirings or electrodes 12 arranged opposite to each other or a change in the distance between the wirings or electrodes, which makes it possible to detect the presence or absence of contact. In particular, the sensitivity at the time of contact can be adjusted by the thickness and conductivity of the conductive gel 40, and the degree of contact can also be detected by monitoring a slight change in the current, the voltage, and/or the electric resistance. In particular, in the living-body contact detection sensor 10 according to the present embodiment, it is possible to maintain the electrical connection between the wirings or electrodes 12 having different polarities via the conductive gel 40, and it is also possible to detect the strength and width when the living body comes into contact by a change in the current, the voltage, and/or the electrical resistance.

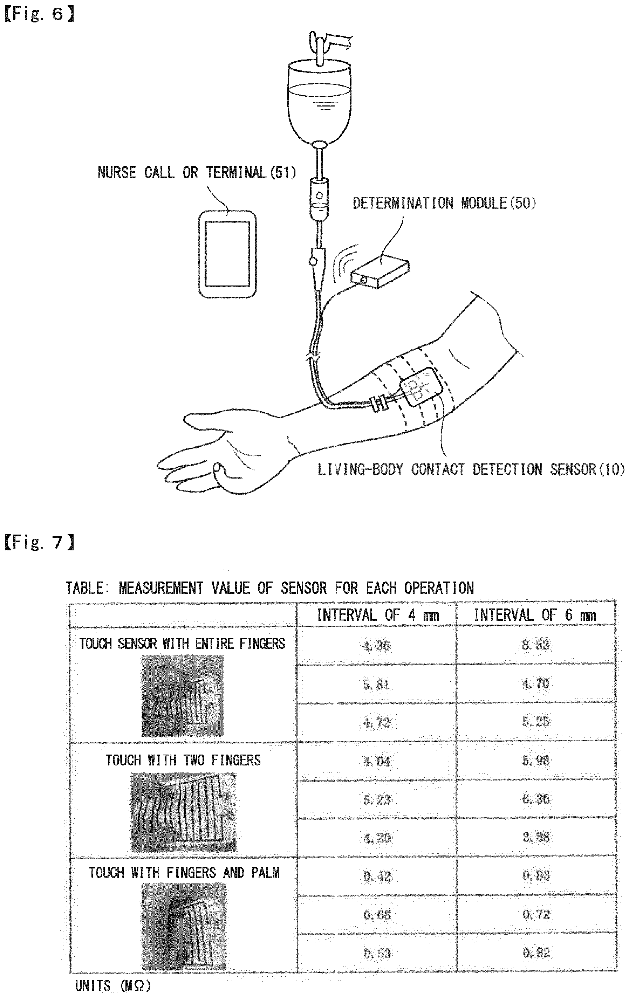

Fig. 6 is a usage state diagram of a body contact detection device 60 configured using the above living-body contact detection sensor 10. The body contact detection device 60 includes: the living-body contact detection sensor 10; and a determination module 50 that determines presence or absence of the contact of the living body based on a change in at least one of a current, a voltage, and an electric resistance acquired from a detection unit 15 in the living-body contact detection sensor 10. In the present embodiment, a connection terminal for connecting the determination module 50 is provided, and the determination module 50 can be connected to the connection terminal.

The determination module 50 can be configured with a determination circuit including an integrated circuit or the like for determining the presence or absence of the contact of the living body based on the change amount and/or the change time of at least one of the current, the voltage, and the electric resistance acquired by the detection unit 15. In particular, with respect to the amount of change and/or the time of change in at least one of the current, the voltage, and the electrical resistance, a certain threshold value can be set, which makes it possible to avoid an erroneous report at the time of simple contact that does not aim to remove the medical catheter or remove the bandage, the covering material, the skin bonding tape, or the like.

That is, the threshold can be set in consideration of a change time of the resistance value of the living-body contact detection sensor 10. In particular, when setting the threshold value, the threshold value regarding the change time of the resistance value and the like can be adjusted depending on the purpose of use (such as drip self-removal prevention, endotracheal intubation tube, bladder indwelling catheter, drainage drain, arterial line, central venous catheter, nasal feeding tube, wound portion, and diaper).

Then, in the determination module 50, in a case where it is determined that the contact of the living body with the detection unit 15 (that is, the contact for removing a medical catheter or removing a bandage, a covering material, or a skin bonding tape), a living-body contact signal indicating that the contact of the living body has occurred is output. The living-body contact signal can be output to a nurse call or an interlocked terminal 51, and an alarm, a warning light, or the like can be provided in the determination module 50. Furthermore, the determination module 50 can be accompanied by a wireless output unit that wirelessly outputs the living-body contact signal. With the wireless output unit, the living-body contact detection device 60 can be used wirelessly, the living-body contact signal can be notified even if a nurse, a caregiver, or the like is not at the bedside, and the action of the patient can be prevented from being restricted even if the living-body contact detection device 60 is used.

By using the living-body contact detection device 60 configured as described above, it is possible to detect in advance intentional removal of a medical catheter by a patient, removal of a bandage, a covering material, a skin bonding tape, or the like, and it is possible to take an appropriate measure also in a medical institution.

In addition, since the living-body contact detection according to the present embodiment is a simple system that detects whether or not there is a contact, it can be effectively used as information for estimating a patient's situation even by a nurse or a caregiver working in a clinical site who is not accustomed to using such a living-body sensor. In addition, in the living-body contact detection device 60, since the detection of self-removal, self-peeling, or the like is more affected by whether or not there is a contact than by the strength of the contact, whether or not the contact of the living body has been made can be detected more accurately and quickly by detecting whether or not there is the contact has been made by a current value, a voltage value, or an electric resistance.

Furthermore, since the living-body contact detection device 60 according to the present embodiment can be retrofitted to the affected part, it is easily introduced without requiring special construction at any clinical site, and furthermore, downsizing and weight reduction are realized. Then, by setting a receiving device (such as a pager type and a tablet type) to be paired with each of living-body contact detection devices 60, it is possible to make the living-body contact detection device 60 usable at home.

In addition, the living-body contact detection sensor 10 described in the above embodiment and the living-body contact detection device 60 using the same can be used in various fields not only for intentional removal of a medical catheter of a patient himself/herself in medical use and removal of a bandage, a covering material, a skin bonding tape, and the like, but also for detecting contact of a living body.

### Example 1

In this experiment, the operation of peeling off the living-body contact detection sensor was performed in three patterns, and a difference in electric resistance value due to a difference in interval (that is, a difference in reaction) was confirmed. In the living-body contact detection sensor used in this experiment, a sheet having a thickness of 0.075 mm was used as a base material. The electrodes were formed by printing carbon. Then, the intervals between the electrodes were set to an interval of 4 mm and an interval of 6 mm, and a change in the electric resistance value was confirmed for each of the intervals. The results are shown in Fig. 7.

In this experiment, similar results were obtained in the living-body contact detection sensor in which the intervals between the electrodes were set to an interval of 4 mm and an interval of 6 mm. Therefore, when the intervals between the electrodes are set to an interval of 4 mm and an interval of 6 mm, it is predicted that there is no difference in detection of the self-removal action.

In addition, in the present example, the time from when the contact of the living-body contact detection sensor was detected to when the living-body contact detection sensor was installed after the bandage was wound around the operation site of the subject was confirmed. As a result, by attaching the detection unit of the living-body contact detection sensor onto a part protected with a bandage, it has been confirmed that it takes 30 seconds or more from when the contact with the detection unit is detected until an operation site which is not desired to be contacted is reached and, for example, the drip needle is started to be pull out. Therefore, a nurse or a caregiver can rush before contacting the operation site or self-removing the drip, and these can be prevented.

In this experiment, the living-body contact detection sensor in which the wirings or electrodes having the thickness of 2.0 mm, with less non-reporting, were formed at intervals of 2.0 mm, has been used, but even in a case where the wirings or electrodes have been formed at other thicknesses or intervals, the living-body contact detection sensor with less non-reporting can be obtained by adjusting the threshold value for determining the contact of the living body. In addition, the threshold can be set by the number of times of detection per unit time, the detection duration time, transition and change of the resistance value/current value or of the voltage value waveform of the voltage value, and the like.

### Industrial Applicability

The living-body contact detection sensor and the living-body contact detection device using the same of the present disclosure can be used to detect in advance that a patient intentionally removes a medical catheter or removes a bandage, a covering material, or a skin bonding tape in the medical field, and can be used as a sensor for detecting contact of a living body without being limited to the medical field. The invention is as defined by the claims as follows.

### Reference Signs List

- 10: Living-body contact detection sensor
- 11: Base material
- 12: Electrode
- 13: Conductive material layer
- 14: ITO film
- 15: Detection unit
- 20: Shielding member
- 30: Dot spacer
- 40: Conductive gel
- 50: Determination module
- 60: Living-body contact detection device

## Claims

1. A living-body contact detection sensor (10) for detecting contact of a living body, comprising:
a base material (11) having a sheet-like shape; and a detection unit (15) made of a conductive material provided on one surface or both sides of the base material (11), wherein
the detection unit (15) includes one or more pairs of wirings or electrodes (12) having different polarities, and
wherein the wirings or electrodes (12) are energized by contact with a living body and at least one of a current, a voltage, and an electric resistance is changed by the contact of the living body; the living body contact detection sensor (10) being **characterized in that** it further comprises a shielding member (20) made of an insulating material configured to cover the portion where the wirings or electrodes (12) are exposed in the detection unit (15), and the living body contact detection sensor (10) cannot be peeled off unless the shielding member (20) is removed.

2. The living-body contact detection sensor (10) according to claim 1, wherein the detection unit (15) is formed by adjacently arranging or oppositely arranging the one or more pairs of wirings or electrodes (12) having different polarities, and the wirings or electrodes (12) are apart from each other at an interval at which at least one of a current, a voltage, and an electric resistance changes by the contact of the living body.

3. The living-body contact detection sensor (10) according to claim 1 or 2, wherein the detection unit (15) is formed by alternately arranging wirings or electrodes (12) having different polarities at intervals for energization when a living body comes into contact with the detection unit (15), and
the wirings or electrodes (12) expose the conductive material, the living body being able to come into direct contact with the wirings or electrodes (12).

4. The living-body contact detection sensor (10) according to any one of claims 1 to 3, wherein
the base material (11) is formed of a resin sheet with flexibility or fabric, and
the detection unit (15) is formed by applying or printing a conductive material including a conductive paste.

5. The living-body contact detection sensor (10) according to any one of claims 1 to 4, wherein the shielding member (20)covers the detection unit (15).

6. A living-body contact detection device (60) comprising: the living-body contact detection sensor (10) according to any one of claims 1 to 5; and a determination module (50) that determines presence or absence of the contact of the living body based on a change in at least one of a current, a voltage, and an electric resistance acquired by a detection unit (15) in the living-body contact detection sensor (10).

7. The living-body contact detection device (60) according to claim 6, wherein the determination module (50) outputs a living-body contact signal indicating that the contact of the living body has occurred in a case where a change amount and/or a change time of at least one of the current, the voltage, and the electric resistance acquired by the detection unit (15) exceeds a preset threshold value.

8. A living-body contact detection method for detecting contact of a living body, comprising:
providing a medical holding device configured to hold a medical device, a medical material, or the like in a living body in a target area where the contact of the living body is detected in a wearer; and installing a living-body contact detection sensor (10) configured to detect the contact of a living body on the medical holding device, wherein the living-body contact detection sensor (10) is the living-body contact detection sensor (10) according to any one of claims 1 to 5.

9. The living-body contact detection method according to claim 8, wherein the medical holding device is a bandage or an adhesive tape.

10. The living-body contact detection method according to claim 8, further comprising:
preventing self-removal of a medical attachment member at an installation region of which there is provided said medical holding device in advance by detecting the contact of the living body with the living-body contact detection sensor (10).

11. The living-body contact detection method according to claim 10, wherein the medical attachment member is a medical catheter or a covering material.

## Patentansprüche

1. Lebendkörperkontakt-Erfassungssensor (10), umfassend:
ein Basismaterial (11), das eine plattenartige Form aufweist; und eine Erfassungseinheit (15), die aus einem leitenden Material gefertigt ist, das auf einer Oberfläche oder beiden Seiten des Basismaterials (11) bereitgestellt ist, wobei
die Erfassungseinheit (15) ein oder mehrere Paare von Drähten oder Elektroden (12) umfasst, die unterschiedliche Polaritäten aufweisen, und
wobei die Drähte oder Elektroden (12) bei Kontakt mit einem lebenden Körper elektrisch geladen werden und dabei mindestens Strom, Spannung oder elektrischer Widerstand verändert werden; wobei der Lebendkörperkontakt-Erfassungssensor (10) **dadurch gekennzeichnet ist, dass** er ferner ein Abschirmelement (20) umfasst, das aus einem isolierenden Material gefertigt ist und konfiguriert ist, um den Abschnitt abzudecken, an dem die Drähte oder Elektroden (12) in der Erfassungseinheit (15) freiliegen,
und der Lebendkörperkontakt-Erfassungssensor (10) nur abgezogen werden kann, wenn das Abschirmungselement (20) entfernt wird.

2. Lebendkörperkontakt-Erfassungssensor (10) nach Anspruch 1, wobei die Erfassungseinheit (15) dadurch gebildet wird, dass ein oder mehrere Paare von Drähten oder Elektroden (12) mit unterschiedlichen Polaritäten nebeneinander oder gegenüberliegend angeordnet sind und die Drähte oder Elektroden (12) in einem Abstand zueinander angeordnet sind, bei dem sich durch den Kontakt des lebenden Körpers mindestens eine der Größen Strom, Spannung oder elektrischer Widerstand ändert.

3. Lebendkörperkontakt-Erfassungssensor (10) nach Anspruch 1 oder 2, wobei die Erfassungseinheit (15) dadurch gebildet wird, dass Drähte oder Elektroden (12) mit unterschiedlichen Polaritäten in regelmäßigen Abständen angeordnet sind und bei Berührung der Erfassungseinheit (15) mit einem lebenden Körper zur Erregung beaufschlagt werden, und
die Drähte oder Elektroden (12) das leitende Material freilegen, wodurch der menschliche Körper in der Lage ist, direkt mit den Drähten oder Elektroden (12) in Kontakt zu kommen.

4. Lebendkörperkontakt-Erfassungssensor (10) nach einem der Ansprüche 1 bis 3, wobei
das Basismaterial (11) aus einer biegsamen Harzplatte oder einem Gewebe gebildet ist, und
die Erfassungseinheit (15) durch Aufbringen oder Drucken eines leitenden Materials, einschließlich einer leitenden Paste, gebildet wird.

5. Lebendkörperkontakt-Erfassungssensor (10) nach einem der Ansprüche 1 bis 4, wobei das Abschirmelement (20) die Erfassungseinheit (15) abdeckt.

6. Lebendkörperkontakt-Erfassungsvorrichtung (60), umfassend: den Lebendkörperkontakt-Erfassungssensor (10) nach einem der Ansprüche 1 bis 5; und ein Bestimmungsmodul (50), das das Vorhandensein oder Nichtvorhandensein des Kontakts mit dem lebenden Körper basierend auf einer Änderung von mindestens einem von einem Strom, einer Spannung und einem elektrischen Widerstand bestimmt, die von einer Erfassungseinheit (15) in dem Lebendkörperkontakt-Erfassungssensor (10) erfasst werden.

7. Lebendkörperkontakt-Erfassungsvorrichtung (60) nach Anspruch 6, wobei das Bestimmungsmodul (50) ein Lebendkörperkontakt-Signal ausgibt, das angibt, dass der Kontakt des lebenden Körpers in einem Fall aufgetreten ist, in dem ein Änderungsbetrag und/oder eine Änderungszeit von mindestens einem von dem Strom, der Spannung und dem elektrischen Widerstand, die von der Detektionseinheit (15) erfasst werden, einen voreingestellten Schwellenwert überschreitet.

8. Lebendkörperkontakt-Erfassungsverfahren zum Erfassen eines Kontaks eines lebenden Körpers, umfassend:
Bereitstellen einer medizinischen Haltevorrichtung, die konfiguriert ist, um eine medizinische Vorrichtung, ein medizinisches Material oder dergleichen in einem lebenden Körper in einem Zielbereich zu halten, in dem der Kontakt des lebenden Körpers bei dem Träger erfasst wird; und installieren eines Lebendkörperkontakt-Erfassungsensors (10), der konfiguriert ist, um den Kontakt eines lebenden Körpers an der medizinischen Haltevorrichtung zu erfassen, wobei der Lebendkörperkontakt-Erfassungsensor (10) der Lebendkörperkontakt-Erfassungsensor (10) nach einem der Ansprüche 1 bis 5 ist.

9. Lebendkörperkontakt-Erfassungsverfahren nach Anspruch 8, wobei die medizinische Haltevorrichtung ein Verband oder ein Klebeband ist.

10. Lebendkörperkontakt-Erfassungsverfahren nach Anspruch 8, ferner umfassend:
Verhindern eines Selbstentfernens eines medizinischen Befestigungselements in einem Installationsbereich, in dem die medizinische Haltevorrichtung bereitgestellt ist, durch das im Voraus erfolgende Erfassen des Kontakts des lebenden Körpers mit dem Lebendkörperkontakt-Erfassungssensor (10).

11. Lebendkörperkontakt-Erfassungsverfahren nach Anspruch 10, wobei das medizinische Befestigungselement ein medizinischer Katheter oder ein Abdeckmaterial ist.

## Revendications

1. Capteur de détection de contact avec un corps vivant (10), comprenant :
un matériau de base (11) ayant la forme d'une feuille ; et une unité de détection (15) constituée d'un matériau conducteur placé sur une surface ou sur les deux côtés du matériau de base (11), dans lequel
l'unité de détection (15) comprend une ou plusieurs paires de fils ou d'électrodes (12) ayant des polarités différentes, et
dans lequel les fils ou les électrodes (12) sont alimentés par le contact avec un corps vivant et au moins l'un d'un courant, d'une tension et d'une résistance électrique est modifié par le contact du corps vivant ; le capteur de détection de contact avec un corps vivant (10) étant **caractérisé en ce qu'**il comprend en outre un élément de blindage (20) constitué d'un matériau isolant configuré pour recouvrir la partie où les fils ou les électrodes (12) sont exposés dans l'unité de détection (15),
et le capteur de détection de contact avec un corps vivant (10) ne peut être décollé que si l'élément de blindage (20) est retiré.

2. Capteur de détection de contact avec un corps vivant (10) selon la revendication 1, dans lequel l'unité de détection (15) est formée par la disposition adjacente ou la disposition opposée des une ou plusieurs paires de fils ou d'électrodes (12) ayant des polarités différentes, et les fils ou les électrodes (12) sont éloignés les uns des autres à un intervalle auquel au moins l'un d'un courant, d'une tension et d'une résistance électrique change par le contact avec le corps vivant.

3. Capteur de détection de contact avec un corps vivant (10) selon la revendication 1 ou 2, dans lequel l'unité de détection (15) est formée par la disposition alternée de fils ou d'électrodes (12) ayant des polarités différentes à intervalles, pour être alimentés lorsqu'un corps vivant entre en contact avec l'unité de détection (15), et
les fils ou les électrodes (12) exposent le matériau conducteur, le corps vivant pouvant entrer en contact direct avec les fils ou les électrodes (12).

4. Capteur de détection de contact avec un corps vivant (10) selon l'une quelconque des revendications 1 à 3, dans lequel
le matériau de base (11) est formé d'une feuille de résine souple ou d'un tissu, et
l'unité de détection (15) est formée par l'application ou l'impression d'un matériau conducteur, notamment une pâte conductrice.

5. Capteur de détection de contact avec un corps vivant (10) selon l'une quelconque des revendications 1 à 4, dans lequel l'élément de blindage (20) recouvre l'unité de détection (15).

6. Dispositif de détection de contact avec un corps vivant (60) comprenant : le capteur de détection de contact avec un corps vivant (10) selon l'une quelconque des revendications 1 à 5 ; et un module de détermination (50) qui détermine la présence ou l'absence du contact avec le corps vivant sur la base d'un changement d'au moins l'un d'un courant, d'une tension ou d'une résistance électrique acquis par une unité de détection (15) dans le capteur de détection de contact avec un corps vivant (10).

7. Dispositif de détection de contact avec un corps vivant (60) selon la revendication 6, dans lequel le module de détermination (50) émet un signal de contact avec un corps vivant indiquant que le contact avec le corps vivant s'est produit dans un cas où un changement de la quantité et/ou un changement du temps d'au moins l'un du courant, de la tension ou de la résistance électrique acquis par l'unité de détection (15) dépasse une valeur de seuil prédéfinie.

8. Procédé de détection de contact avec un corps vivant pour détecter un contact avec un corps vivant, comprenant :
la fourniture d'un dispositif de maintien médical configuré pour maintenir un dispositif médical, un matériel médical ou l'équivalent dans un corps vivant dans une zone cible où le contact avec le corps vivant est détecté chez un porteur ; et l'installation d'un capteur de détection de contact avec un corps vivant (10) configuré pour détecter le contact d'un corps vivant sur le dispositif de maintien médical, dans lequel le capteur de détection de contact avec un corps vivant (10) est le capteur de détection de contact avec un corps vivant (10) selon l'une quelconque des revendications 1 à 5.

9. Procédé de détection de contact avec un corps vivant selon la revendication 8, dans lequel le dispositif de maintien médical est un bandage ou une bande adhésive.

10. Procédé de détection de contact avec un corps vivant selon la revendication 8, comprenant en outre :
la prévention du retrait par un patient d'un élément de fixation médicale dans une zone d'installation où se trouve ledit dispositif de maintien médical, à l'avance, en détectant le contact avec le corps vivant avec le capteur de détection de contact avec un corps vivant (10).

11. Procédé de détection de contact avec un corps vivant selon la revendication 10, dans lequel l'élément de fixation médicale est un cathéter médical ou un matériau de recouvrement.
